Europäisches Patentamt

European Patent Office

Office européen des brevets

(19)

(11)     EP 0 712 641 B1

(12)     **FASCICULE DE BREVET EUROPEEN**

(45) Date de publication et mention
de la délivrance du brevet:
**28.04.2004   Bulletin 2004/18**

(51) Int Cl.⁷: **A61N 1/37**, A61N 1/39

(21) Numéro de dépôt: **95402567.2**

(22) Date de dépôt: **15.11.1995**

(54) **Procédé d'ajustage d'un paramètre électrique d'un appareil médical implantable et dispositif de mise en oeuvre**

Verfahren zum Einstellen eines elektrischen Parameters einer implantierbaren Vorrichtung und Vorrichtung zur Durchführung des Verfahrens

Method for adjusting an electrical parameter of an implantable medical device and apparatus for carrying out the method

(84) Etats contractants désignés:
**BE CH DE ES FR GB IT LI SE**

(30) Priorité: **16.11.1994  FR 9413696**

(43) Date de publication de la demande:
**22.05.1996   Bulletin 1996/21**

(73) Titulaire: **ELA MEDICAL (Société anonyme)**
**92541 Montrouge (FR)**

(72) Inventeurs:
• **Pons, Pascal**
**F-38320 Crolles (FR)**

• **Dal Molin, Renzo**
**F-92320 Chatillon (FR)**

(74) Mandataire: **Dupuis-Latour, Dominique et al**
**SEP Pagenberg & Associés**
**14 boulevard Malesherbes**
**75008 Paris (FR)**

(56) Documents cités:
**EP-A- 0 027 265          EP-A- 0 138 008**
**EP-A- 0 406 830          US-A- 4 761 591**
**US-A- 5 084 685**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

L'invention concerne l'ajustage automatique de certains paramètres électriques d'un dispositif électronique, notamment d'un dispositif médical implantable actif tel qu'un stimulateur ou défibrillateur cardiaque.

À cet égard, bien que dans la suite de la description on fasse principalement référence au cas d'un stimulateur cardiaque, l'invention est applicable de façon générale à une très grande variété de dispositifs électroniques. Elle l'est avantageusement, mais de façon non limitative, aux "dispositifs médicaux implantables actifs" tels que définis par la directive 90/385/CEE du 20 juin 1990 du Conseil des communautés européennes.

L'invention s'applique tout particulièrement à l'ajustage automatique de paramètres électriques de circuits intégrés sur une puce monolithique de composant. En effet, la complexité croissante des dispositifs implantables et les contraintes de miniaturisation rendent indispensable l'utilisation d'un ou plusieurs circuits intégrés. Mais les paramètres technologiques de la fabrication de ces circuits peuvent introduire des dispersions dans les caractéristiques des composants d'un lot de production à un autre ou même d'un circuit à un autre, avec une dispersion corrélative des paramètres électriques des circuits du stimulateur.

Il en est ainsi des fréquences d'horloge des divers oscillateurs du stimulateur, par exemple l'oscillateur qui définit la cadence maximale limite de stimulation, ou l'oscillateur de pilotage du microprocesseur.

Comme autres paramètres électriques sujets à de telles dispersions, on trouve également les caractéristiques fréquentielles (principalement la fréquence de coupure), notamment des divers filtres impliqués dans la détection de l'activité cardiaque, qu'il s'agisse de filtres passifs ou actifs.

L'ajustage de ces divers paramètres implique l'ajustage de la valeur de composants du circuit.

Une première série de techniques employées à cet effet consiste à prévoir des composants passifs extérieurs à la puce, par exemple des éléments résistifs, ajustés par enlèvement de matière au moyen d'un laser ou d'une sableuse. Cette manière de procéder présente comme inconvénients majeurs l'encombrement, la limitation du type de composant ajustable (essentiellement des résistances) et le caractère définitif de l'ajustage (donc l'impossibilité de corriger une éventuelle dérive dans le temps du paramètre qui aura été ajusté).

Lorsqu'il s'agit de composants situés sur la puce, on peut prévoir un ajustage par *bonding* sélectif d'une échelle de composants affectés de valeurs de poids décroissants. Cette technique est cependant délicate à industrialiser, nécessite un nombre important de plages de contact et consomme donc une surface importante de puce ; elle est en outre, comme la précédente, définitive.

Il en est de même de la technique de commutation dite *Zener zapping*, qui présente les mêmes inconvénients d'irréversibilité.

Une seconde série de techniques consiste à réaliser l'ajustage automatique des paramètres par voie logicielle, en conservant les valeurs de consigne dans une mémoire électronique utilisée pour corriger les paramètres électriques des circuits sujets à dispersion. Ces techniques, malgré leur avantage de permettre une modification ultérieure de l'ajustage, présentent cependant les inconvénients d'accroissement notable de la complexité de conception du circuit et de sensibilité aux baisses d'alimentation (mémoires RAM) et aux effets des radiations qu'elles peuvent recevoir (mémoires RAM ou E$^2$PROM).

L'un des buts de l'invention est de remédier à ces divers inconvénients en proposant un procédé et un dispositif permettant un ajustage des paramètres électriques d'un dispositif électronique, notamment d'un dispositif médical implantable actif, ajustage qui soit opéré directement sur des composants et de façon à la fois automatique et réversible.

Plus précisément, comme on l'exposera par la suite, l'ajustage automatique selon l'invention présente les avantages suivants, qui n'avaient jamais pu être rassemblés jusqu'à présent par les techniques connues :

- le dispositif d'ajustage est complètement intégré à la puce, ce qui contribue à un encombrement minimal et une utilisation maximale de la surface de celle-ci ;
- cette mise en oeuvre n'entraîne pas d'accroissement sensible de la consommation, paramètre toujours important dans le cas des dispositifs implantables actifs, où il est nécessaire de préserver la durée de vie de la pile par tous les moyens possibles ;
- l'automaticité de l'ajustage assure une très grande fiabilité et une très grande stabilité dans le temps, un contrôle étant possible à tout moment de la vie du dispositif implantable ;
- l'ajustage peut prendre en compte non seulement les dispersions intrinsèques aux composants du circuit, mais également celles résultant de l'environnement dans lequel celui-ci fonctionne (notamment les conditions de température) ;
- l'ajustage peut être facilement inhibé par télémétrie, par exemple à des fins de test ou de caractérisation, et être restauré par la suite : comme on le verra, il est possible, par exemple lors d'une reprogrammation totale du dispositif implantable, de désactiver et réactiver à la demande l'auto-ajustage au moyen du programmateur.

On verra également que l'ajustage automatique selon l'invention peut s'appliquer aussi bien à des fréquences

**EP 0 712 641 B1**

d'horloge d'oscillateur qu'à des bandes passantes de filtres avec, dans l'un et l'autre cas, des circuits respectifs comportant un très grand nombre de composants semblables en commun, simplifiant d'autant la conception d'ensemble de la puce du dispositif implantable.

Le US-A-5 084 685 décrit un procédé comportant les étapes consistant à : a) produire une oscillation dont la période est fonction de la valeur d'un composant ajustable par commutation sélective d'une pluralité de composants additionnels, ledit paramètre électrique à ajuster étant lui-même fonction de la valeur de ce composant ajustable et l'état des commutations des composants additionnels étant déterminé par une valeur numérique conservée dans un registre de mémoire ; b) mesurer le rapport entre la période produite à l'étape (a) et une période de référence de valeur fixe connue ; c) déterminer l'écart entre le rapport mesuré à l'étape (b) et une valeur de consigne prédéterminée ; d) modifier la valeur numérique conservée dans le registre jusqu'à trouver une valeur minimisant l'écart déterminé à l'étape (c) ; et e) verrouiller le registre sur la valeur numérique finale obtenue à l'étape (d).

Mais dans ce procédé connu, la valeur finale du registre est une valeur recalculée, ce qui implique la mise en oeuvre de moyens de calcul numérique, typiquement à microprocesseur, difficiles à incorporer à un dispositif implanté, tant pour des raisons de complexité que de consommation. Pour pallier cet inconvénient, selon l'invention, à l'étape (b), la détermination dudit rapport est effectuée par comptage du nombre de périodes de l'oscillation produite à l'étape (a) pendant la durée d'une période de référence ou, inversement, par comptage du nombre de périodes de référence pendant la durée d'une période de l'oscillation produite à l'étape (a) ; à l'étape (c), la détermination dudit écart est effectuée par comparaison du nombre ainsi compté avec la valeur de consigne; et à l'étape (d), ladite valeur numérique est modifiée par incrémentation ou par décrémentation à partir d'une valeur minimale ou maximale, respectivement, et la minimisation dudit écart est considérée comme atteinte lorsqu'un incrément supplémentaire de la valeur numérique provoque un changement de signe de cet écart.

Dans une première forme de mise en oeuvre du procédé, ledit paramètre électrique à ajuster est une fréquence d'horloge et l'oscillation produite à l'étape (a) est l'oscillation de cette horloge.

Dans une seconde forme de mise en oeuvre du procédé, ledit paramètre à ajuster est une fréquence de coupure de filtre et l'oscillation produite à l'étape (a) est une oscillation produite soit en commutant le composant ajustable dans un circuit oscillant pendant la durée de la phase d'ajustage, soit en commutant un composant apparié au composant ajustable à ajuster et appartenant à un circuit oscillant distinct du filtre à ajuster.

Avantageusement, la valeur numérique du registre de mémoire peut être forcée à une valeur de sécurité prédéterminée sur commande interne ou externe du dispositif.

L'invention concerne également un dispositif électronique, notamment un dispositif médical implantable actif, mettant en oeuvre les différents aspects du procédé ci-dessus. En particulier, l'invention permet d'intégrer sur une même puce monolithique le composant ajustable avec ses composants additionnels et ses moyens de commutation sélective associés, le registre de mémoire, les moyens de mesure du rapport entre périodes, les moyens déterminateurs d'écart et les moyens modificateurs de la valeur numérique.

D'autres caractéristiques et avantages de l'invention apparaîtront à la lecture de la description détaillée ci-dessous de deux modes de réalisation détaillés de l'invention, faite en référence aux dessins annexés.

La figure 1 est un schéma par blocs illustrant un circuit d'ajustage automatique d'une fréquence d'oscillateur selon l'invention.

La figure 2 est un schéma par blocs illustrant un circuit d'ajustage automatique de la fréquence de coupure d'un filtre du premier ordre selon l'invention.

La figure 3 illustre le signal oscillatoire produit par le circuit de la figure 2 pendant la phase d'ajustage du filtre.

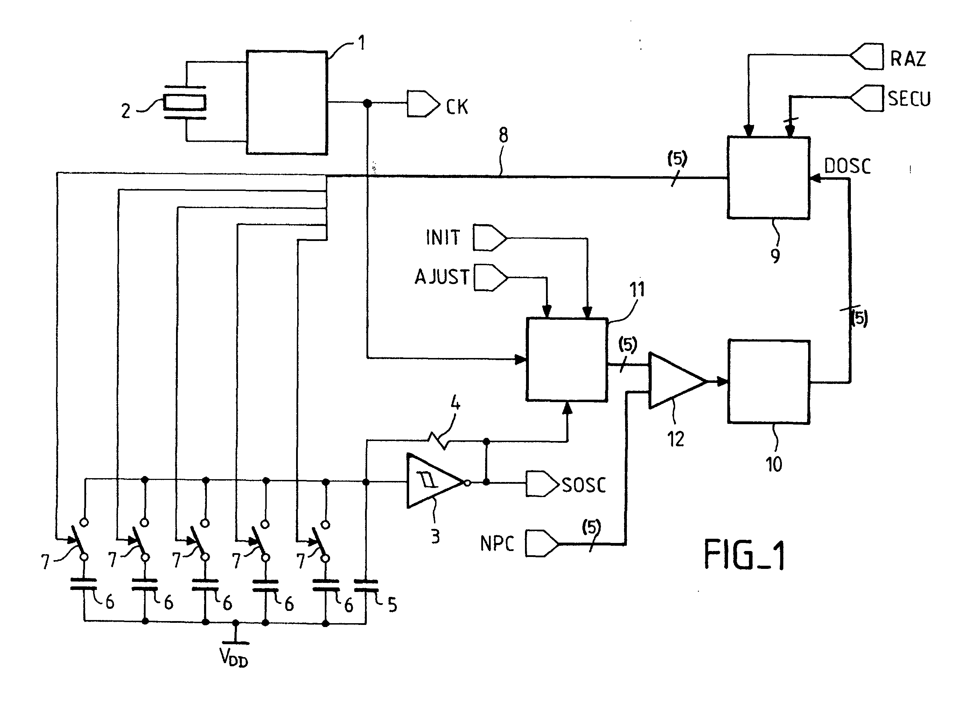

La figure 1 illustre l'exemple d'un oscillateur RC intégré dont on souhaite ajuster la fréquence.

Le principe de base consiste à comparer la fréquence de l'oscillateur à une fréquence de référence, qui est celle d'un oscillateur à quartz présent dans le dispositif implantable.

Selon les cas, la fréquence à ajuster peut être inférieure ou supérieure à celle de l'oscillateur de référence à quartz.

Le premier cas, où la fréquence de l'oscillateur à ajuster est inférieure à celle de l'oscillateur à quartz, est par exemple celui de l'oscillateur qui définit la limite supérieure du rythme cardiaque (généralement d'environ 190 coups par minute), déterminée par un oscillateur de 1 kHz alors que la fréquence de l'oscillateur à quartz est de 32 kHz.

Dans ce cas, on compare la période de l'oscillateur à ajuster à un nombre prédéfini de périodes de l'oscillateur de référence. On compte le nombre de périodes (ou le nombre de transitions) de l'oscillateur de référence contenues dans une période, ou un multiple d'une période, de l'oscillateur à ajuster, on compare ce nombre à une valeur de consigne et on ajuste par itérations successives un composant du circuit de manière à réduire au minimum l'écart entre le nombre compté et la valeur de consigne.

Le second cas, où la fréquence de l'oscillateur à ajuster est supérieure à celle de l'oscillateur à quartz, correspond par exemple à celui de l'oscillateur du microprocesseur, généralement de 500 kHz alors que l'oscillateur à quartz n'a qu'une fréquence de 32 kHz.

Dans ce dernier cas on procède de façon inverse, c'est-à-dire que l'on compte le nombre de périodes (ou de

3

transitions) de l'oscillateur à ajuster contenues dans une période, ou un multiple d'une période, de l'oscillateur de référence, l'ajustage étant ensuite réalisé de la même manière.

L'ajustage de la fréquence de fonctionnement d'un oscillateur RC peut être obtenue de diverses manières.

Dans l'exemple qui va suivre, ceci est réalisé par ajustage de la capacité, de la manière que l'on exposera en détail. Cette possibilité n'est cependant pas la seule, et d'autres mises en oeuvre équivalentes sont envisageables, notamment l'ajustage de la résistance (bien que cette manière de procéder soit plus délicate du fait de la résistance propre $R_{on}$ des transistors de commutation) ou encore la commutation de sources de courant, la capacité de l'oscillateur étant alors chargée à courant constant.

Plus précisément, on a représenté en 1 l'oscillateur de référence, qui est piloté par un quartz 2, dont on pourra considérer que la fréquence est parfaitement stable. Cet oscillateur produit un signal de référence CK.

L'oscillateur RC est réalisé autour d'une bascule *(trigger)* 3, avec un élément résistif 4, fixe dans cet exemple, monté entre son entrée et sa sortie, et un condensateur 5 monté entre son entrée et la source de potentiel $V_{DD}$. Le signal résultant, dont la fréquence sera à ajuster, a été désigné SOSC.

Pour réaliser cet ajustement, on prévoit une échelle de capacités d'ajustement supplémentaires 6 qui peuvent être sélectivement et séparément commutées en parallèle sur la capacité principale 5 au moyen de commutateurs statiques 7, par exemple des MOS commandés chacun par un signal de tension appliqué sur l'une des lignes correspondantes d'un bus 8, qui est un bus de cinq bits correspondant aux cinq capacités qu'il est possible de commuter.

Les capacités 6 sont dans des rapports 1/2, 1/4, 1/8 et 1/16 par rapport à la capacité de plus forte valeur, de manière à permettre un ajustement par choix d'une parmi seize combinaisons possibles par pas de capacité constants.

Les signaux du bus 8 assurant la commande des commutateurs 7 proviennent d'un registre 9 qui comporte une entrée RAZ de remise à zéro initiale, une entrée SECU de forçage d'un code de sécurité sur cinq bits et une entrée DOSC représentant un code d'ajustage.

Ce code d'ajustage DOSC ("données oscillateur") est un code sur cinq bits engendré par un circuit 10 permettant de générer successivement tous les codes de cinq bits possibles, du plus bas au plus haut.

Ce circuit 10 est commandé par un compteur 11 recevant d'une part la fréquence de référence CK et d'autre part la fréquence à ajuster SOSC ; ce compteur 11 peut être réinitialisé par une impulsion INIT et rendu actif par un signal AJUST appliqué pendant toute la durée de l'opération d'auto-ajustage.

Comme, dans le présent exemple, la fréquence de l'oscillateur à ajuster est supérieure à celle de l'oscillateur à quartz, le compteur 11 compte le nombre de périodes de l'oscillateur à quartz CK contenu dans une période de l'oscillateur à ajuster SOSC. Le nombre ainsi déterminé, qui est un mot de cinq bits, est appliqué sur l'une des entrées d'un comparateur 12 dont l'autre entrée reçoit la valeur de consigne NPC (nombre de périodes à compter).

On va maintenant exposer la manière dont l'auto-ajustage est mis en oeuvre dans ce circuit.

L'opération commence par une remise à zéro du registre 9 (RAZ = '1'), qui fait généralement suite à une initialisation complète du dispositif implantable, demandée au moyen d'un programmateur par liaison télémétrique.

Le dispositif implantable est alors en mode de "veille", de manière à garantir que l'auto-ajustage s'effectue toujours dans des conditions normales, avec l'oscillateur à quartz opérationnel et tous les autres paramètres électriques à leur valeur nominale.

Dans ce mode, le système force automatiquement la valeur du registre 9 à un code d'ajustage de sécurité (valeur SECU), ce qui assure au dispositif une fréquence de fonctionnement "moyenne", imprécise mais satisfaisante pour assurer une sécurité minimale.

On commande alors le dispositif implantable pour passer du mode "veille" au mode "nominal".

On commence alors l'opération d'ajustage, en positionnant à '1' le code AJUST, ce qui rend opérationnel le compteur 11 et donc également le comparateur 12 et le générateur de codes d'ajustage 10 situé en aval de ce dernier.

Le générateur 10 applique alors la première valeur possible du code DOSC, par exemple la valeur '11111' correspondant à la fermeture de tous les commutateurs 7. Le circuit va alors osciller à sa fréquence la plus basse.

Sur un front montant de l'oscillateur de référence (signal CK), on initialise le compteur par une impulsion INIT = '1' de courte durée, puis on procède au comptage proprement dit du nombre de périodes de l'oscillateur à ajuster (nombre de périodes du signal SOSC).

Sur le front montant suivant de CK, le comptage cesse et le comparateur 12 confronte le contenu du compteur 11 à la valeur de consigne NPC.

Si le nombre de périodes comptées est inférieur à la valeur de consigne, on commande le générateur 10 de manière qu'il décrémente d'une unité la valeur du code d'ajustage DOSC (qui passe par exemple à '11110') et on répète le cycle : sur le front montant suivant de CK, on initialise le compteur 11, on compte de nombre des périodes du signal SOSC, etc.

Lorsque le nombre de périodes comptées devient supérieur ou égal à la valeur de consigne NPC, ceci signifie que l'on a atteint l'optimum, c'est-à-dire l'écart minimal entre le nombre de périodes réel et le nombre de périodes de consigne. On considère alors que l'oscillateur est ajusté au mieux et l'on désactive l'auto-ajustage (AJUST = '0').

Le dispositif implantable peut alors fonctionner, en mode nominal, avec l'oscillateur ajusté sur la dernière valeur du mot d'ajustage DOSC, valeur qui reste mémorisée dans le registre 9.

On notera qu'en cas d'incident la valeur du registre 9 sera automatiquement forcée à la valeur de sécurité SECU permettant au dispositif implantable de fonctionner convenablement, bien que de façon non optimale, en toutes circonstances.

Le tableau I donne un exemple de paramètres électriques obtenus avec un circuit du type de celui de la figure 1, réalisé conformément aux enseignements de l'invention.

Tableau I

| | |
|---|---|
| Fréquence nominale de l'oscillateur RC ($F_{osc}$) | 500 kHz |
| Dispersion-type $F_{osc}$ avant ajustage | 303 kHz < $F_{osc}$ < 825 kHz |
| Soit en pourcentage | -39 % / +65 % |
| Fréquence de l'oscillateur à quartz ($F_q$) | 32,768 kHz |
| Nombre de périodes à compter | 30 |
| Erreur d'échantillonnage : au maximum 1 période sur 30 | < 3,3 % |
| Nombre de bits (Nb) du code d'ajustage DOSC | 5 |
| Erreur maximale d'ajustage : $(\Delta T_{osc}/T_{osc})/(2^{Nb}-1)$ | ± 2,1 % |
| Dispersion-type $F_{osc}$ après ajustage | 481 kHz < $F_{osc}$ < 519 kHz |

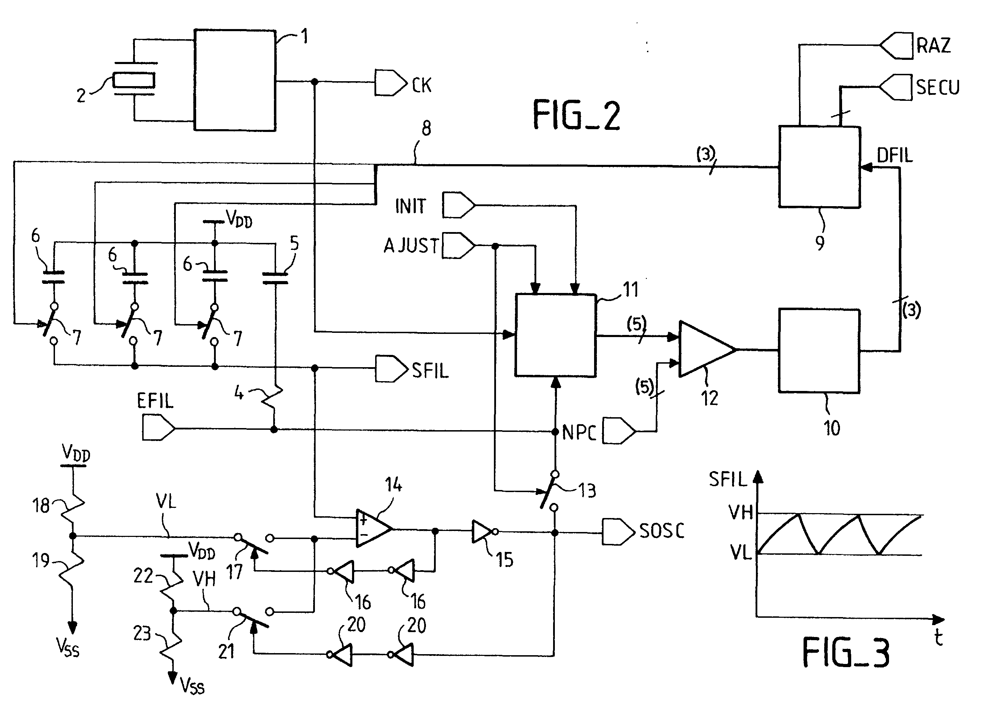

La figure 2 illustre un autre circuit à auto-ajustage selon l'invention, qui est ici un filtre continu RC du premier ordre.

Dans ce circuit, on introduit temporairement le filtre dans un circuit oscillant, on ajuste la fréquence de ce dernier de la même manière que pour le mode de réalisation précédent (par commutation de capacités), puis on restitue le circuit RC à sa fonction de filtre une fois l'ajustement réalisé.

Cette manière de procéder n'est cependant pas la seule possible, et l'on pourrait par exemple envisager de dupliquer tous les composants capacitifs du filtre, une série de composants constituant le filtre proprement dit et l'autre série servant à faire osciller un circuit RC distinct, fonctionnant de façon permanente. En effet, dans le domaine de la microélectronique, en présence de deux éléments (tels que des capacités) définis de la même façon à proximité les uns des autres, on constate un excellent appariement de sorte que, si l'on ajuste l'oscillateur par une combinaison appropriée de capacités commutées, on peut estimer que la capacité obtenue sur le filtre par la même combinaison de commutations sera pratiquement identique.

En variante, au lieu de procéder à cette duplication pour la totalité des séries de capacités, on peut prévoir de réaliser une série étalon de capacités commune à tous les filtres utilisant les mêmes valeurs de composants, et servant à piloter un oscillateur RC ; l'ajustage de l'oscillateur étalon permet d'obtenir ainsi l'ajustage de tous les filtres associés en appliquant à tous les circuits la même combinaison de commutations de capacités.

Le circuit de la figure 2 correspond au cas où l'on introduit temporairement le filtre dans un circuit oscillant. Un certain nombre d'éléments étant communs aux circuits des figures 1 et 2, ils seront dès lors affectés des mêmes références numériques ; dans la mesure où ces éléments fonctionnent de façon semblable, ils ne seront pas décrits plus en détail et l'on pourra se référer à la description qui en a été déjà donnée plus haut.

Ainsi, le filtre RC comporte un élément résistif fixe 4, une capacité 5, également fixe, et une série de capacités commutables d'ajustement 6, par exemple au nombre de trois, commutables sélectivement et séparément par des commutateurs statiques 7 en fonction d'un mot de trois bits appliqué sur un bus 8 et provenant d'un registre 9.

Ce filtre RC constitué des éléments 4, 5 et 6 est relié à une entrée EFIL et à une sortie SFIL.

Pour ajuster ce filtre, on l'utilise temporairement pour déterminer la fréquence d'un oscillateur, fréquence que l'on ajustera de la même manière que pour l'oscillateur proprement dit de la figure 1, au moyen des éléments 7, 8, 9, 10, 11 et 12 qui jouent le même rôle que dans le cas de la figure 1.

Pour réaliser cette commutation temporaire, on prévoir un commutateur 13 qui sera fermé lorsque la commande d'ajustage AJUST sera appliquée.

La fermeture de ce commutateur appliquera en entrée du compteur 11 le signal de sortie SOSC d'un oscillateur constitué d'un amplificateur opérationnel 14 dont une première entrée est reliée au point commun de la résistance 4 et des condensateurs 5 et 6 (point commun correspondant à la sortie de filtre SFIL) et dont la sortie est reliée à l'entrée du compteur 11 via un inverseur 15 et l'interrupteur 13.

La sortie de l'amplificateur 14 commande également, via deux inverseurs 16 montés en cascade, un commutateur 17 qui applique à la seconde entrée de l'amplificateur opérationnel 14 une tension de référence basse VL obtenue par exemple au moyen d'un pont diviseur formé de deux résistances 18 et 19 montées entre le potentiel positif d'alimentation $V_{DD}$ et le potentiel négatif d'alimentation $V_{SS}$.

La sortie de l'inverseur 15 commande par ailleurs, via deux inverseurs 20 montés en cascade, un commutateur 21 permettant d'appliquer à la seconde entrée de l'amplificateur opérationnel 14 une tension de référence haute VH, obtenue par exemple au moyen d'un pont diviseur formé de deux résistances 22 et 23 montées entre $V_{DD}$ et $V_{SS}$. On notera que les commandes des interrupteurs 17 et 21 étant toujours inversées, ceux-ci ne seront jamais simultanément fermés.

Avec cette configuration, le signal de sortie SFIL va donc alterner périodiquement entre le niveau haut VH et le niveau bas VL (comme illustré figure 3), les commutateurs 17 et 21 basculant à chaque franchissement du seuil VH ou VL et faisant passer le circuit RC d'un cycle de charge à un cycle de décharge, ou inversement.

Avec ce principe d'oscillateur, la période des oscillations est donnée par la relation suivante ($R_1$, $R_2$, $R_3$ et $R_4$ étant les valeurs des résistances 22, 23, 18 et 19, respectivement) :

$$F_{osc} = 1 / (RC \, \text{Log} \, [(R_2/R_1).(R_3/R_4)]),$$

Cette période est donc indépendante des tensions $V_{DD}$ et $V_{SS}$ et ne dépend que de l'élément résistif 4, des éléments capacitifs 5 et 6 et des rapports des résistances 18, 19 et 22, 23, qui peuvent être très bien appariées dans une technologie de circuit intégré.

On utilise ainsi les éléments résistif 4 et capacitifs 5, 6 du filtre pour contrôler un oscillateur (signal SOSC), dont l'ajustement sera réalisé de la même manière que dans le cas de la figure 1.

La fréquence de coupure $F_{coupure}$ étant déterminée par la relation :

$$F_{coupure} = 1 / (2\pi RC),$$

il vient :

$$F_{coupure} / F_{osc} = (\text{Log} \, [(R_2/R_1).(R_3/R_4)]) / (2\pi) = \text{constante}$$

L'ajustement de $F_{osc}$ entraînera donc l'ajustement parfaitement homothétique de $F_{coupure}$. Une fois l'ajustement obtenu, le signal AJUST est supprimé, ce qui a pour effet d'ouvrir le commutateur 13 et donc de restituer le circuit RC que l'on a ajusté à sa fonction normale de filtre, plus aucun autre signal n'étant appliqué en entrée de ce filtre que le signal à filtrer EFIL.

Le tableau II donne un exemple de paramètres électriques obtenus avec un circuit du type de celui de la figure 2, réalisé conformément aux enseignements de l'invention.

Tableau II

| | |
|---|---|
| Fréquence de coupure nominale ($F_{coupure}$) | 250 Hz |
| Dispersion-type $F_{coupure}$ avant ajustage | 173 Hz < $F_{coupure}$ < 391 Hz |
| Soit en pourcentage | -31 % / +56 % |
| Fréquence nominale de l'oscillateur RC ($F_{osc}$) | 1024 Hz |
| Fréquence de l'oscillateur à quartz ($F_q$) | 32,768 kHz |
| Nombre de périodes à compter | 32 |
| Erreur d'échantillonage : au maximum 1 période sur 32 | < 3,1 % |
| Nombre de bits du code d'ajustage DFIL (Nb) | 3 |
| Erreur maximale d'ajustage : $(\Delta T_{osc}/T_{osc}) / (2^{Nb}-1)$ | ± 5,7 % |
| Dispersion-type $F_{coupure}$ après ajustage | 236 Hz < $F_{coupure}$ < 265 Hz |
| Soit en pourcentage | -5,4 % / +6,1 % |

On peut étendre le procédé d'auto-ajustage de l'invention à d'autres paramètres du circuit électrique, comme la fréquence de résonance du circuit de télémétrie, qui est composé d'une auto-inductance et d'une capacité rapportée sur le circuit, et dont on peut ajuster la valeur. Dans ce cas, l'oscillation n'est pas entretenue ; on compte le nombre d'oscillations de l'oscillateur à quartz 2 entre deux passages à zéro de la tension mesurée aux bornes du circuit résonant.

**Revendications**

1. Un procédé d'ajustage d'un paramètre électrique d'un dispositif électronique, notamment d'un dispositif médical implantable actif tel qu'un stimulateur ou défibrillateur cardiaque, comprenant les étapes consistant à :

   a) produire une oscillation dont la période est fonction de la valeur d'un composant ajustable par commutation sélective d'une pluralité de composants additionnels, ledit paramètre électrique à ajuster étant lui-même fonction de la valeur de ce composant ajustable et l'état des commutations des composants additionnels étant déterminé par une valeur numérique conservée dans un registre de mémoire ;
   b) mesurer le rapport entre la période produite à l'étape a) et une période de référence de valeur fixe connue ;
   c) déterminer l'écart entre le rapport mesuré à l'étape b) et une valeur de consigne prédéterminée ;
   d) modifier la valeur numérique conservée dans le registre jusqu'à trouver une valeur minimisant l'écart déterminé à l'étape c) ; et
   e) verrouiller le registre sur la valeur numérique finale obtenue à l'étape d),

   procédé **caractérisé en ce que** :

   - à l'étape b), la détermination dudit rapport est effectuée par comptage du nombre de périodes de l'oscillation produite à l'étape a) pendant la durée d'une période de référence ou, inversement, par comptage du nombre de périodes de référence pendant la durée d'une période de l'oscillation produite à l'étape a) ;
   - à l'étape c), la détermination dudit écart est effectuée par comparaison du nombre ainsi compté avec la valeur de consigne ; et
   - à l'étape d), ladite valeur numérique est modifiée par incrémentation ou par décrémentation à partir d'une valeur minimale ou maximale, respectivement, et la minimisation dudit écart est considérée comme atteinte lorsqu'un incrément supplémentaire de la valeur numérique provoque un changement de signe de cet écart.

2. Le procédé de la revendication 1 dans lequel ledit paramètre électrique à ajuster est une fréquence d'horloge et l'oscillation produite à l'étape a) est l'oscillation de cette horloge.

3. Le procédé de la revendication 1 dans lequel ledit paramètre à ajuster est une fréquence de coupure de filtre et l'oscillation produite à l'étape a) est une oscillation produite en commutant le composant ajustable dans un circuit oscillant pendant la durée de la phase d'ajustage.

4. Le procédé de la revendication 1 dans lequel ledit paramètre à ajuster est une fréquence de coupure de filtre et l'oscillation produite à l'étape a) est une oscillation produite en commutant un composant apparié au composant ajustable à ajuster et appartenant à un circuit oscillant distinct du filtre à ajuster.

5. Le procédé de la revendication 1 dans lequel la valeur numérique du registre de mémoire peut être forcée à une valeur de sécurité prédéterminée sur commande interne ou externe du dispositif implantable.

6. Un dispositif électronique, notamment un dispositif médical implantable actif tel qu'un stimulateur ou défibrillateur cardiaque, avec au moins un paramètre électrique ajustable, comprenant :

   — un circuit oscillateur (3-6 ; 4-6, 14-23) comprenant un composant ajustable (5, 6) déterminant sa période d'oscillation, ce composant comprenant une pluralité de composants additionnels (6) et des moyens (7, 8) de commutation sélective de ces composants additionnels ;
   — un registre de mémoire (9) conservant une valeur numérique déterminant l'état des commutations des composants additionnels ;
   — des moyens (11) de mesure du rapport entre la période (SOSC) produite par le circuit oscillateur et une période (CK) de référence de valeur fixe connue ;
   — des moyens (12) pour déterminer l'écart entre le rapport mesuré par les moyens de mesure et une valeur de consigne prédéterminée (NPC) ; et
   — des moyens (10) pour modifier la valeur numérique conservée dans le registre jusqu'à trouver une valeur minimisant l'écart déterminé par les moyens déterminateurs d'écart,

   dispositif **caractérisé en ce que** :

   — les moyens (11) de mesure du rapport entre périodes comprennent un compteur du nombre de périodes du circuit oscillateur pendant la durée d'une période de référence ou, inversement, du nombre de périodes de référence pendant la durée d'une période du circuit oscillateur ;

— les moyens (12) déterminateurs d'écart comprennent un comparateur du nombre ainsi compté avec la valeur de consigne ; et

— les moyens (10) modificateurs de la valeur numérique conservée dans le registre comprennent un circuit d'incrémentation ou de décrémentation de ce registre à partir d'une valeur minimale ou maximale, respectivement, cette incrémentation ou décrémentation étant arrêtée lorsqu'un incrément supplémentaire de la valeur numérique provoque un changement de signe dudit écart.

7. Le dispositif de la revendication 6 dans lequel ledit paramètre électrique à ajuster est une fréquence d'horloge et le circuit oscillateur est celui de cette horloge.

8. Le dispositif de la revendication 6 dans lequel ledit paramètre à ajuster est une fréquence de coupure de filtre et le circuit oscillateur est formé par commutation du composant ajustable du filtre dans ce circuit oscillateur pendant la durée de la phase d'ajustage.

9. Le dispositif de la revendication 6 dans lequel ledit paramètre à ajuster est une fréquence de coupure de filtre et le circuit oscillateur est distinct du filtre à ajuster et comporte un composant apparié au composant ajustable du filtre.

10. Le dispositif de la revendication 6 dans lequel la valeur numérique du registre de mémoire (9) peut être forcée à une valeur de sécurité prédéterminée (SECU) sur commande interne ou externe du dispositif.

11. Le dispositif de la revendication 6 dans lequel le composant ajustable (5, 6) avec ses composants additionnels (6) et ses moyens (7) de commutation sélective associés, le registre de mémoire (9), les moyens (11) de mesure du rapport entre périodes, les moyens (12) déterminateurs d'écart et les moyens (10) modificateurs de la valeur numérique sont des éléments intégrés sur une même puce monolithique.

**Claims**

1. A method for adjusting an electrical parameter of an electronic device, in particular an active implantable medical device such as a pacemaker or cardiac defibrillator, comprising the steps of:

   a) producing an oscillation the period of which is a function of the value of an adjustable component by selectively switching a plurality of additional components, said electrical parameter to be adjusted being itself a function of the value of said adjustable component and the switching state of the additional components being determined by a digital value stored in a memory register;
   b) measuring the ratio between the period produced at step a) and a reference period having a fixed known value;
   c) determining the difference between the ratio measured at step b) and a predetermined set value;
   d) modifying the digital value stored in the register until finding a value which minimises the difference determined in step c); and
   e) latching the register to the final digital value obtained in step d),

   said method being **characterised in that**:

   in step b), the determination of said ratio is made by counting the number
   of periods of the oscillation produced at step a) for the duration of a reference period or, conversely, by counting the number of reference periods for the duration of an oscillation produced at step a);
   in step c), the determination of said difference made by comparing the number thus counted with the set value; and
   in step d), said digital value is modified by incrementing or decrementing from a minimal or maximal value, respectively, and the minimisation of said difference is considered as reached when an additional increment of the digital value induces a change of sign of said difference.

2. The method of claim 1, wherein said electrical parameter to be adjusted is a clock frequency and the oscillation produced in step a) is the oscillation of said clock.

3. The method of claim 1, wherein said parameter to be adjusted is a filter cutoff frequency and the oscillation produced in step a) is an oscillation produced by switching the adjustable component in an oscillating circuit for the duration of the adjustment step.

4. The method of claim 1, wherein said electrical parameter to be adjusted is a filter cutoff frequency and the oscillation produced in step a) is an oscillation produced by switching a component matched to the adjustable component to be adjusted and being a part of an oscillating circuit which is separate from the filter to be adjusted.

5. The method of claim 1, wherein the digital value of the memory register may be forced to a predetermined safety value upon internal or external control of the implantable device.

6. An electronic device, in particular an active implantable medical device such as a pacemaker or cardiac defibrillator, with at least one adjustable electrical parameter, comprising:

   - an oscillator circuit (3-6; 4-6; 14-23) including an adjustable components (5, 6) determining its oscillation period, said component including a plurality of additional components (6) and means (7, 8) for selective switching of said additional components;
   - a memory register (9) storing a digital value determining the switching state of the additional components;
   - means (11) for measuring the ratio between the period (COSC) produced by the oscillator circuit and a reference period (CK) having a fixed known value;
   - means (12) for determining the difference between the ratio measured by the measuring means and a predetermined set value (NPC);
   - means (10) for modifying the digital value stored in the register until finding a value which minimises the difference determined by the difference determining means;

   said device being **characterised in that**:

   - the means (11) for measuring the ratio between the periods includes a counter of the number of periods of the oscillation circuit for the duration of a reference period or, conversely, of the number of reference periods for the duration of a period of the oscillation circuit;
   - the means (12) for determining the difference includes a comparator of the number thus counted with the set value; and
   - the means (10) for modifying the digital value stored in the register includes a circuit for the incrementation or the decrementation of said register from a minimal or maximal value, respectively, said incrementation or decrementation being stopped when an additional increment of the digital value induces a change of sign of said difference.

7. The device of claim 6, wherein said electrical parameter to be adjusted is a clock frequency and the oscillator circuit is the circuit of said clock.

8. The device of claim 6, wherein said parameter to be adjusted is a filter cutoff frequency and the oscillator circuit is obtained by switching the adjustable component of the filter in said oscillator circuit for the duration of the adjustment step.

9. The device of claim 6, wherein said electrical parameter to be adjusted is a filter cutoff frequency and the oscillator circuit is separate from the filter to be adjusted and includes a component matched to the adjustable component of the filter.

10. The device of claim 6, wherein the digital value of the memory register (9) may be forced to a predetermined safety value (SECU) upon internal or external control of the implantable device.

11. The device of claim 6, wherein the adjustable component (5, 6) along with its additional components (6) and its associated selective switching means (7), the memory register (9), the means (11) for measuring the ratio between the periods, the means (12) for determining the difference , and the means (10) for modifying the digital value are elements which are integrated on a same monolithic chip.

**Patentansprüche**

1.  Verfahren zur Anpassung eines elektrischen Parameters einer elektronischen Vorrichtung, insbesondere einer aktiven implantierbaren medizinischen Vorrichtung wie ein Herzschrittmacher oder Defibrillator, umfassend die Schritte bestehend aus:

    a) Erzeugen einer Oszillation, deren Periode abhängig ist von dem Wert eines Bestandteils, der einstellbar ist durch selektive Kommutation einer Vielzahl von zusätzlichen Bestandteilen, wobei der einzustellende elektrische Parameter selbst abhängig ist vom Wert dieses einstellbaren Bestandteils, und der Zustand der Kommutationen der zusätzlichen Bestandteile bestimmt wird durch einen numerischen Wert, der in einem Speicherregister gehalten wird;
    b) Messen des Verhältnisses zwischen der in Schritt a) erzeugten Periode und einer Referenzperiode von bekanntem festem Wert;
    c) Bestimmen des Unterschieds zwischen dem in Schritt b) gemessenen Verhältnis und einem vorbestimmten Schwellwert;
    d) Modifizieren des in dem Register gespeicherten numerischen Werts, bis ein Wert gefunden wird, der den in Schritt c) bestimmten Unterschied minimiert; und
    e) Sperren des Registers bei dem finalen numerischen Wert, der in Schritt d) erhalten wurde,

    wobei das Verfahren **dadurch gekennzeichnet ist, dass**:

    -   in Schritt b) die Bestimmung des Verhältnisses bewirkt wird durch Zählen der Anzahl der Perioden der in Schritt a) erzeugten Oszillation während der Dauer einer Referenzperiode, oder umgekehrt, durch Zählen der Anzahl der Referenzperioden während der Dauer einer Periode der in Schritt a) erzeugten Oszillation;
    -   in Schritt c) die Bestimmung des Unterschieds bewirkt wird durch Vergleich der so gezählten Anzahl mit dem Schwellwert; und
    -   in Schritt d) der numerische Wert durch Inkrementieren oder durch Dekrementieren modifiziert wird, jeweils ausgehend von einem minimalen oder maximalen Wert, und die Minimierung des Unterschieds als erreicht angesehen wird, wenn eine zusätzliche Inkrementierung des numerischen Wertes eine Änderung des Vorzeichens dieses Unterschieds bewirkt.

2.  Verfahren nach Anspruch 1, in welchem der einzustellende elektrische Parameter eine Taktfrequenz ist und die in Schritt a) erzeugte Oszillation die Oszillation dieses Taktes ist.

3.  Verfahren nach Anspruch 1, in welchem der einzustellende Parameter eine Grenzfrequenz eines Filters ist und die in Schritt a) erzeugte Oszillation eine Oszillation ist, die durch Vertauschen des einstellbaren Bestandteils in einem oszillierenden Schaltkreis während der Dauer der Einstellphase ist.

4.  Verfahren nach Anspruch 1, in welchem der einzustellende Parameter eine Grenzfrequenz eines Filters ist und die in Schritt a) erzeugte Oszillation eine Oszillation ist, die erzeugt wird durch Kommutationen des einzustellenden einstellbaren Bestandteils mit einem passenden Bestandteil und zu einem oszillierenden Schaltkreis gehört, der verschieden ist vom einzustellenden Filter.

5.  Verfahren nach Anspruch 1, in welchem der numerische Wert des Speicherregisters auf einen Sicherheitswert gesetzt oder gezwungen werden kann, welcher vorherbestimmt ist durch innere oder äußere Steuerung der implantierbaren Vorrichtung.

6.  Elektronische Vorrichtung, insbesondere aktive implantierbare medizinische Vorrichtung wie ein Herzschrittmacher oder Defribrillator, mit mindestens einem einstellbaren elektrischen Parameter, umfassend:

    -   einen Oszillatorschaltkreis (3-6; 4-6; 14-23), umfassend einen einstellbaren Bestandteil (5, 6), welcher seine Oszillationsperiode bestimmt, wobei der Bestandteil eine Vielzahl von zusätzlichen Bestandteilen (6) aufweist und Mittel (7, 8) zur selektiven Vertauschung dieser zusätzlichen Bestandteile;
    -   ein Speicherregister (9), welches einen numerischen Wert speichert, der den Zustand der Vertauschungen der zusätzlichen Bestandteile bestimmt;
    -   Mittel (11) zur Messung des Verhältnisses zwischen der Periode (SOSC), die durch den Oszillatorschaltkreis erzeugt wird, und einer Referenzperiode (CK) von festem bekanntem Wert;
    -   Mittel (12) zum Bestimmen des Unterschieds zwischen dem durch Messmittel gemessenen Verhältnis und einem vorbestimmten Schwellwert (NPC); und

- Mittel (10) zum Modifizieren des numerischen Wertes, der im Register gehalten wird, bis ein Wert gefunden wird, der den durch die Unterschiedsbestimmungsmittel bestimmten Unterschied minimiert;

wobei die Vorrichtung **dadurch gekennzeichnet, dass**:

- die Mittel (11) zum Messen des Verhältnisses zwischen Perioden einen Zähler der Anzahl der Perioden des Oszillatorschaltkreises während der Dauer einer Referenzperiode, oder umgekehrt der Anzahl der Referenzperioden während der Dauer einer Periode des Oszillatorschaltkreises, enthalten;
- die Mittel (12) zur Bestimmung des Unterschieds einen Komparator der so gezählten Anzahl mit dem Schwellwert umfassen; und
- die Mittel (10) zur Modifikation des numerischen Wertes, der im Register gehalten wird, einen Inkrementierungs- oder Dekrementierungsschaltkreis dieses Registers umfassen, jeweils ausgehend von einem minimalen oder maximalen Wert, wobei die Inkrementierung oder Dekrementierung angehalten wird, wenn eine zusätzliche Inkrementierung des numerischen Wertes eine Änderung des Vorzeichens des Unterschieds bewirkt.

7. Vorrichtung nach Anspruch 6, bei welcher der einzustellende elektrische Parameter eine Taktfrequenz ist und der Oszillatorschaltkreis derjenige des Takts ist.

8. Vorrichtung nach Anspruch 6, bei welcher der einzustellende Parameter eine Grenzfrequenz eines Filters ist und der Oszillatorschaltkreis durch Kommutation des einstellbaren Bestandteils des Filters im Oszillatorschaltkreis während der Dauer der Einstellphase ist

9. Vorrichtung nach Anspruch 6, bei welcher der einzustellende Parameter eine Grenzfrequenz eines Filters ist und der Oszillatorschaltkreis verschieden vom einzustellenden Filter ist und einen Bestandteil aufweist, der zum einstellbaren Bestandteil des Filters gehört.

10. Vorrichtung nach Anspruch 6, bei welcher der numerische Wert des Speicherregisters (9) auf einen vorbestimmten Sicherheitswert (SECU) gesetzt oder gezwungen werden kann, auf interne oder externe Steuerung der Vorrichtung.

11. Vorrichtung nach Anspruch 6, bei welcher der einstellbare Bestandteil (5, 6) mit seinen zusätzlichen Bestandteilen (6) und seinen zugehörigen Mitteln (7) zur selektiven Kommutation, das Speicherregister (9), die Mittel (11) zur Messung des Verhältnisses zwischen Perioden, die Mittel (12) zur Bestimmung des Unterschieds und die Mittel (10), die den numerischen Wert modifizieren, Elemente sind, die auf dem gleichen monolithischen Chip integriert sind.

FIG_1

EP 0 712 641 B1

FIG_2

FIG_3

EP 0 712 641 B1